# FASCICULE DE BREVET EUROPEEN

(11) **EP 0 593 520 B1**
(45) Date de publication et mention de la délivrance du brevet: **29.09.1999**
(21) Numéro de dépôt: 92912769.4
(22) Date de dépôt: 18.06.1992
(51) Int. Cl.: A61K 31/56, A61K 31/57, C07J 9/00

(54) **COMPOSITIONS OCULAIRES CONTENANT DES STEROIDES ET LEUR UTILISATION POUR LE TRAITEMENT DU GLAUCOME**
STEROIDE ENTHALTENDE ZUSAMMENSETZUNGEN ZUR ANWENDUNG AM AUGE UND IHRE VERWENDUNG ZUR BEHANDLUNG DES GLAUKOMS
OCULAR COMPOSITIONS CONTAINING STEROIDS AND THEIR USE IN TREATING GLAUCOMA

(30) Priorité: 18.06.1991 FR 9107412; 17.07.1991 FR 9109015; 17.07.1991 FR 9109016; 17.07.1991 FR 9109017; 09.08.1991 FR 9110160
(43) Date de publication de la demande: 27.04.1994
(73) Titulaire: LABORATOIRE THERAMEX S.A., 98000 Monaco (MC)
(72) Inventeur: SCHWADROHN, Gérard, F-06000 Nice (FR)
(74) Mandataire: Burtin, Jean-François
(86) Numéro de dépôt international: FR9200549
(87) Numéro de publication internationale: WO9222300

(56) Documents cités:
- EP-A- 0 250 088
- EP-A- 0 371 617
- WO-A-86/02554
- WO-A-87/07141
- WO-A-89/03839
- WO-A-90/12027
- FR-A- 2 271 833
- US-A- 4 383 992
- Archives of Ophthalmology, vol. 101, no. 4, avril 1983, American Medical Association, K. PHILLIPS et al.: "Effects of prednisolone and medroxyprogesterone on corneal wound healing, ulceration, and neovascularization", pages 640-643, voir abrégé; page 641, paragraphe 3
- Il Farmaco, vol. 31, no. 12, décembre 1976, P. DE RUGGIERI et al.: "Structure-activity relationship of some steroidal compounds in ocular inflammation", pages 849-855, voir page 851, tableaux I,VI,VII; page 852, paragraphe 3
- Experimental Eye Research, vol. 26, no. 6, juin 1978, Academic Press, (London, GB), J.W. LAMBLE et al.: "Some effects of progestogens, oestrogens and androgens on the ocular tension of rabbits and owl monkeys", pages 599-610, voir le document en entier

## Description

L' invention se rapporte au domaine de la chimie thérapeutique et particulièrement au domaine de la pharmacotechnie.

Elle a spécifiquement pour objet l' utilisation pour la réalisation d'une conposition pharmaceutique destinée au traitement du glaucome d'au moins un composé de structure stéroïdienne choisi dans le groupe constitué par :
A - LES 19-NOR PREGNA 4,6-DIENES DE FORMULE GENERALE I 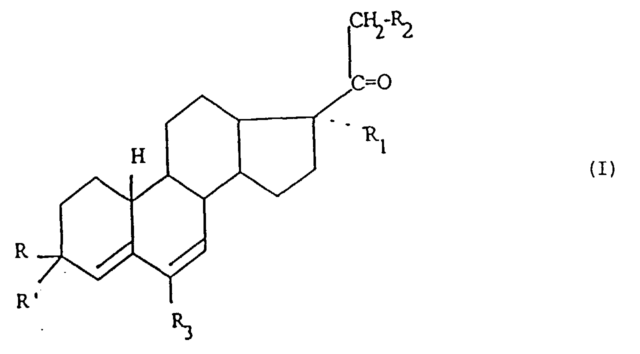 dans laquelle
   R₁ représente un radical hydroxy, alcoxy, acyloxy
   R₂ représente de l'hydrogène ou un radical alcoyle inférieur ayant de 1 à 4 atomes de carbone
   R et R' ensemble forment un groupement cétonique, hydroxyimino, alcoxy imino ou acyloxy imino
   ou bien séparément, lorsque R = H, R' est un groupement hydroxy, alcoxy ou acyloxy,
   et R₃ représente un radical alcoyle inférieur ayant de 1 à 3 atomes de carbone,
B - LES 19-NOR PREGNA 4,6-DIENES DE FORMULE GENERALE II 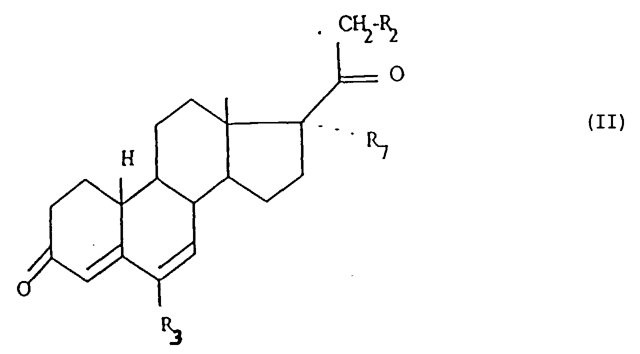 dans laquelle
   R₂ représente un atome d'hydrogène ou un radical alcoyle ayant de 1 à 6 atomes de carbone, en chaîne droite ou ramifiée
   R₇ représente un radical alcoyle inférieur, identique ou différent de R₂, ayant de 1 à 6 atomes de carbone, en chaîne droite ou ramifiée
   et R₃ est défini comme précédemment,
C - LES COMPOSES DE STRUCTURE 19-NOR PREGNENIQUE DE FORMULE GENERALE III 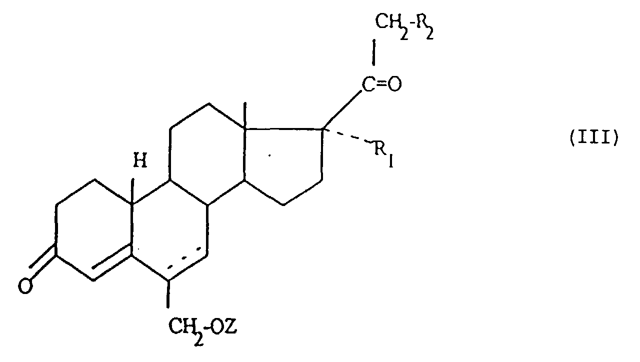 dans laquelle
   R₁ représente un radical hydroxy, alcoxy, acyloxy ou alcoyle ayant de 1 à 4 atomes de carbone
   R₂ représente de l'hydrogène ou un radical alcoyle inférieur en chaîne droite ou ramifiée ayant de 1 à 4 atomes de carbone
   Z représente de l'hydrogène, un radical alcoyle ou un radical acyle
   et le trait pointillé symbolise une double liaison carbone-carbone éventuelle,
D - LES DERIVES 19-NOR PREGNENIQUES DE FORMULE GENERALE IV 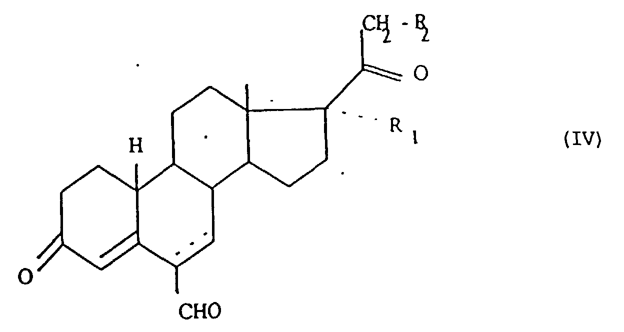 dans laquelle
   R₁ représente un radical hydroxy, alcoxy, acyloxy ou alcoyle ayant de 1 à 4 atomes de carbone
   R₂ représente de l'hydrogène ou un radical alcoyle inférieur, en chaîne droite ou ramifiée, ayant de 1 à 4 atomes de carbone
   et le trait pointillé symbolise une double liaison carbone-carbone éventuelle,
E - LES DERIVES 6-HALOGENOMETHYL 19-NOR PREGNENIQUE DE FORMULE GENERALE V 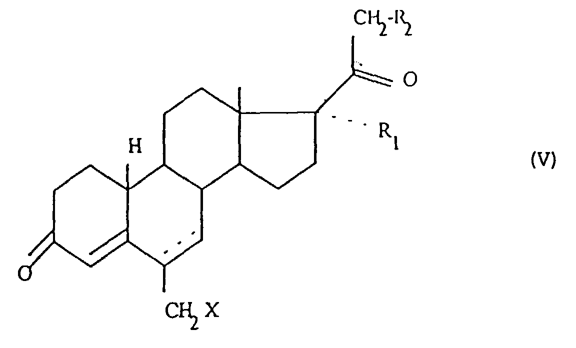 dans laquelle
   X représente un atome d'halogène
   R₁ et R₂ sont définis comme précédemment
   et le trait pointillé symbolise une double liaison carbone-carbone éventuelle,
F - LES COMPOSES DE STRUCTURE 3,20-DIOXO Δ4-PREGNENIQUE REPONDANT A LA FORMULE GENERALE VI 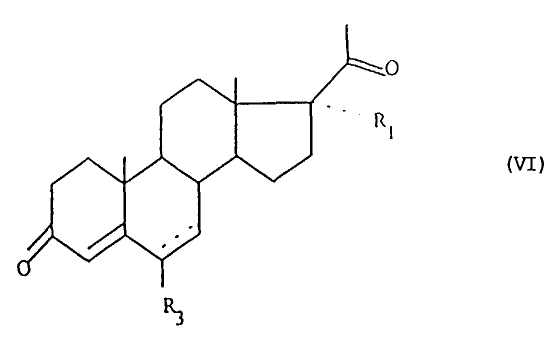 dans laquelle
   R₁ représente un hydroxyle libre, éthérifié ou estérifié ou un radical alcoyle inférieur
   R₃ représente de l'hydrogène ou un radical alcoyle inférieur ayant de 1 à 4 atomes de carbone
   et le trait pointillé symbolise une double liaison carbone-carbone éventuelle,
G - LES COMPOSES 11,18-EPOXYANDROSTENIQUES DE FORMULE GENERALE VII 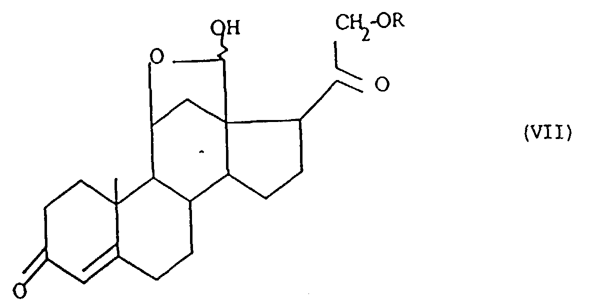
   dans laquelle le trait ondulé symbolise une orientation α ou β
   et R représente de l'hydrogène ou un radical acyle dérivé d'un acide organique carboxylique ayant de 1 à 10 atomes de carbone.

Parmi les composés de formule générale I, on pourra citer tout spécialement les dérivés de 17α-hydroxy 3,20-dioxo 19-nor pregna 4,6-diène de formule générale Ia 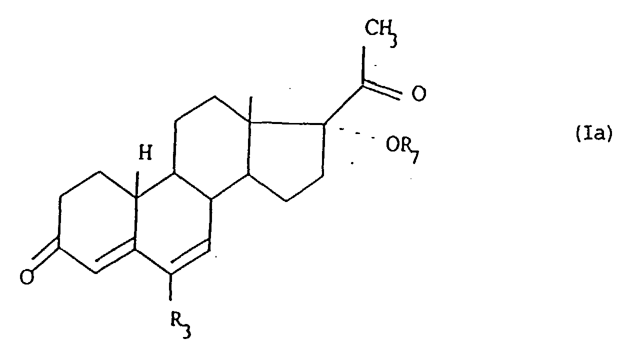
dans laquelle R₇ représente un atome d'hydrogène, un radical acyle ayant de 1 à 8 atomes de carbone
et R₃ est un radical méthyle ou propyle.

Parmi les composés de formule générale III, on pourra citer tout spécialement :
a) les dérivés de 17α-hydroxy 3,20-dioxo 19-nor pregna 4,6-diène de formule générale IIIa 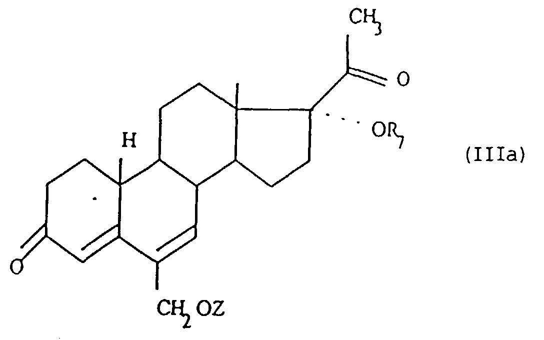 dans laquelle
   R₇ représente un atome d'hydrogène, un radical alcoyle ayant de 1 a 8 atomes de carbone, un radical tétrahydropyranyl-2 ou un radical acyle dérivé d'un acide carboxylique aliphatique, saturé ou non saturé, ayant de 1 à 10 atomes de carbone, éventuellement substitué par un radical aryle ou cycloalcoyle
   Z représente de l'hydrogène, un radical alcoyle ou un radical acyle,
b) les dérivés 17α-hydroxy 3,20-dioxo 21-méthyl 19-nor pregna 4,6-diène de formule générale IIIb 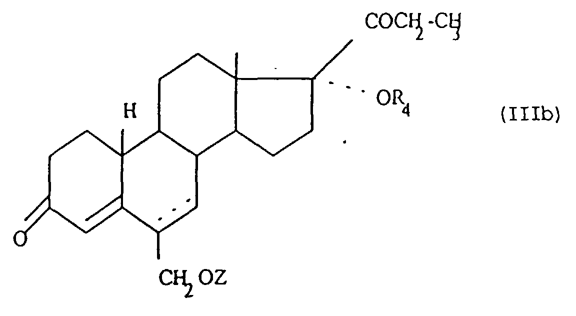 dans laquelle
   R₄ représente un atome d'hydrogène, un radical alcoyle inférieur, un radical méthoxyméthyle, un radical tétrahydropyranyl-2 ou un radical acyle d'un acide organique carboxylique ayant de 1 à 10 atomes de carbone,
   Z représente de l'hydrogène, un radical alcoyle ou un radical acyle,
c) les dérivés alcoylés de 3,20-dioxo 19-nor pregna 4,6-diène de formule générale IIIc 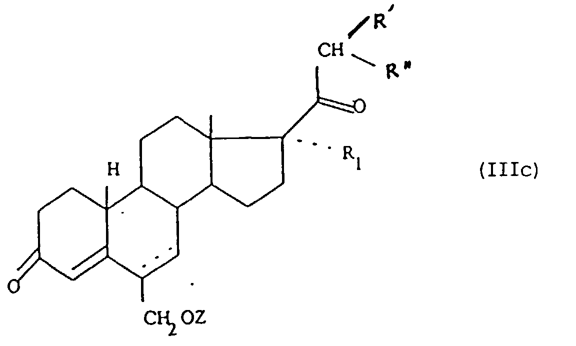 dans laquelle
   R' et R'' représentent chacun un atome d'hydrogène ou un radical alcoyle ayant de 1 à 3 atomes de carbone, en chaîne linéaire ou ramifiée
   R₁ représente un radical alcoyle inférieur, identique ou différent de R' et R'', ayant de 1 à 3 atomes de carbone en chaîne linéaire ou ramifiée
   Z représente de l'hydrogène, un radical alcoyle ou un radical acyle,
d) les dérivés 19-nor pregna 4-éniques de formule générale IIId 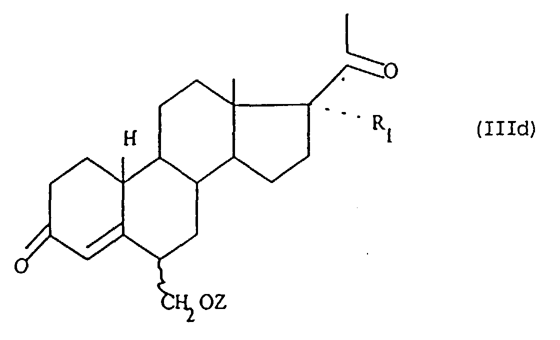 dans laquelle
   R₁ est un radical alcoyle, un hydroxy, un alcoxy, un acyloxy
   Z représente de l'hydrogène, un radical alcoyle ou un radical acyle
   et le trait ondulé indique une orientation α ou β.

Les composés IIIa, IIIb, IIIc et IIId sont décrits dans la demande de brevet française n° 91.09097 déposée au nom de la société demanderesse, le 18 juillet 1991.

Parmi les composés de formule générale IIIa, on citera plus particulièrement le 6-pivaloyloxy méthyl 17α-hydroxy 3,20-dioxo 19-nor pregna 4,6-diène, le 6-pivaloyloxy méthyl 17α-acetoxy 3,20-dioxo 19-nor pregna 4,6-diène et, le 6-hydroxyméthyl 17α-acétoxy 3,20-dioxo 19-nor pregna 4,6-diène.

Parmi les composés de formule générale V, on citera tout particulièrement :
- le 3,20-dioxo 17α-acetoxy 6-chlorométhyl 19-nor pregna 4,6-diène de formule Va 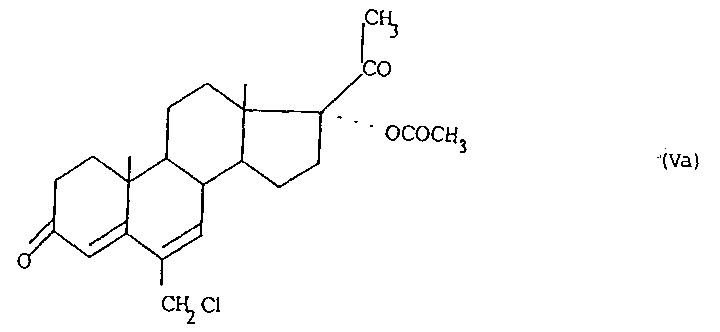
- et le 3,20-dioxo 17α-acetoxy 6-fluorométhyl 19-nor pregna 4,6-diène de formule Vb

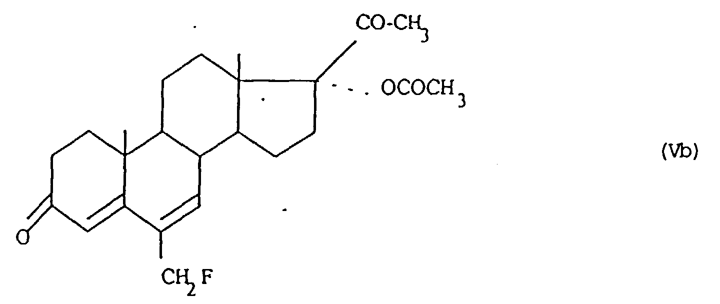

Parmi les composés de formule générale VI, on citera en particulier la 17α-hydroxy progestérone.

Parmi les composés de formule générale VII, on citera en particulier l'isoaldostérone de formule 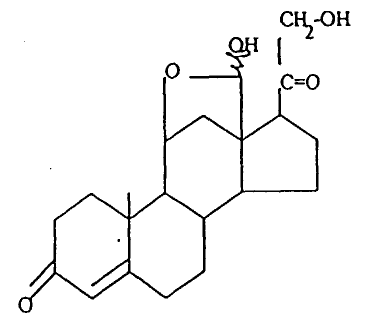 ainsi que ses esters alipahatiques ou aromatiques.

A cette fin, les compositions pharmaceutiques sont, selon l'invention, de préférence présentées sous forme solide à dissolution extemporanée semi-solide ou liquide. Les composés de formule I, II, III, IV, V, VI ou VII se trouvent sous forme de suspension ou dissouts en solution, répartis en flacons, tubes ou systèmes unidoses.

Les compositions pharmaceutiques ainsi obtenues sont additionnées, si nécessaire, d'agents isotonisants pour atteindre l'isotonie aux sécrétions lacrymales, d'agents de stabilisation ou de conservation et/ou d'agents anti-oxydants.

Comme agent isotonisant on pourra citer des sels minéraux ou des dérivés organiques.

Comme agent de stabilisation, on citera plus particulièrement les agents chelatants comme l'acide éthylène diaminotétraacétique et ses sels. Comme agents anti-oxydants, on citera l'acide ascorbique, les métabisulfites de métaux alcalins, les hypophosphites de métaux alcalins, le salicylate de sodium, le gentisate de sodium ou le gallate d'isopropyle.

Dans ce qui précède et sauf indication contraire, un radical alcoyle inférieur possède de 1 à 6 atomes de carbone comme un radical méthyle, éthyle, isopropyle, terbutyle, isobutyle, n-pentyle, néopentyle ou nhéxyle.

Un halogène est un atome de fluor ou de chlore.

Un reste acyle d'acide organique carboxylique renferme de 1 à 10 atomes dans la partie carbonée comme par exemple un acétyle, un propionyle, un hexanoyle, un benzoyle, un dichoroacétyle, un vanilloyle, un isovanilloyle, un triméthoxy benzoyle, un naphtoyle α ou β, un furoyle, un nicotinoyle ou un thenoyle.

Un reste acyle d'un acide alcoyl carbonique contient de 1 à 8 atomes de carbone dans la chaine alcoyle comme par exemple un cycloalcoylméthyl carbonique ou un radical cycloalcoyl propyl carbonique.

La nouvelle utilisation selon l'invention s' adresse au traitement des hypertonies intra-oculaires et de la maladie glaucomateuse. Les compositions pharmaceutiques produites renferment de 0,05 à 1 % de principe actif stéroïdien. Elles sont réalisées par mise en solution ou mise en suspension d'un principe actif de formule générale I à VII dans un solvant aqueux approprié.

On sait qu'il existe trois types de glaucome :
1. les glaucomes primitifs qui représentent 86% de tous les glaucomes. On en distingue deux sortes qui sont tout particulièrement importants :
   - le glaucome à angle ouvert (GA0)
   - le glaucome par fermeture de l'angle (GFA)
2. les glaucomes secondaires
3. les glaucomes congénitaux

Les signes cliniques de ces affections sont ceux d'une neuropathie optique avec :
- baisse de l'acuité visuelle
- déficit du champ visuel
- altération des fibres du nerf optique aboutissant progressivement à la cécité.

Une pression intra-oculaire (PI0) supérieure à 15 mmHg ± 2.5 mmHg est observée dans la majorité des cas.

L'ensemble des traitements actuels visent à normaliser cette PI0 qui est le signe le plus immédiatement quantifiable et garant de la bonne efficacité du traitement.

Les traitements actuels locaux possèdent des effets pharmacologiques analogues à ceux des hormones du système nerveux autonome, qu'ils soient sympathomimétiques ou para-sympathomimétiques (adrénaline, épinéphrine, pilocarpine ..).

La découverte durant ces dix dernières années de l'action de produits β-bloquants a encore renforcé l'importance des systèmes sympatho-et para-sympathomimétiques au niveau oculaire.

Les traitements généraux (per os ou en perfusion) utilisés, visent comme les traitements locaux à faire diminuer la formation de l'humeur aqueuse (exception faite pour la pilocarpine).

Tous ces traitements comportent des inconvénients qui rendent nécessaire une surveillance médicale régulière. L'usage de ces produits présente des contre-indications nombreuses et importantes :
- les β-bloquants (asthme bronchique, insuffisance cardio-vasculaire, etc...)
- les antiglaucomateux sympathomimétiques (le glaucome à angle fermé)
- les antiglaucomateux para-sympathomimétiques (les iridocyclites) et de nombreux effets indésirables (réactions cornéo-conjonctivales, mydriase ou myosis iriens, sécheresse oculaire, modification du champ visuel, effets cardiovasculaires, effets respiratoires généraux,. dermatologiques ..) variant suivant la nature du produit.

Les médicaments produits selon l' invention visent à améliorer cette situation. Ils sont d'une efficacité au moins égale, mais surtout leur tolérance et leur innocuité aux doses testées sont sensiblement meilleures. De plus, ils s'adressent indifféremment aux glaucomes à angles ouvert et aux glaucomes par fermeture de l'angle et ne présentent pratiquement aucun des effets indésirables et des contre-indications sus-mentionnés.

Des études déjà anciennes avaient déjà tenté de démontrer le rôle régulateur sur la PI0 de l'oeil normal, de la progestérone. Cette dernière et même l'hormone du corps jaune avaient déjà été utilisées. On a constaté que ces produits abaissaient la pression intra-oculaire d'une façon objective mais surtout éphémère, effet que les auteurs ont attribué à l'action diurétique des agents progestatifs, à l'action para-sympathicotrope de la lutéine qui diminue chez le rat la mydriase due à l'atropine et enfin à l'effet antagoniste de l'action hypotensive sur l'oeil, de la folliculine (cf. Ärztlich Wochenschrift 5 (1950) 34). Plus récemment, TZU LUNG CHIANG, (Prostaglandins 4, 3 (1973) 415), a montré que la progestérone inhibait la réponse hypertensive de l'oeil à la perfusion intra-veineuse de PGA₂. Toutefois, pour réaliser un effet significatif quoique peu durable, les doses de progestérone administrées ont été considérables (25 mg/kg). Le même auteur (Europ. J. of Pharmacology 22 (1973) 304) a confirmé que la progestérone antagonisait l'augmentation de la pression intra-oculaire.

Les effets de la progestérone paraissent résulter d'une synergie avec l'effet de l'épinéphrine ou de l'éphédrine, administrée localement.

Les conclusions des auteurs sont que l'efficacité du traitement contre l' hypertension intra-oculaire à l'aide de la progestérone en réponse aux prostaglandines était limitée. Il faut une dose très élevée de progestérone administrée fréquemment. Selon ces mêmes auteurs, l'action de la progestérone résulterait d' une synergie avec les catécholamines circulantes.

Par ailleurs, la PGE, administrée en perfusion veineuse inhibe complètement l'augmentation du niveau de production des protéines dans l'humeur aqueuse.

En outre, cette action oculaire des progestomimétiques semble indépendante des propriétés connues de la progestérone et surtout sans corrélation avec un effet progestomimétique. C'est pourquoi, l'efficacité des médicaments produits selon l' invention n'est pas en relation avec le niveau d'affinité connu du principe actif pour le récepteur à la progestérone ou avec le niveau d'activité progestéronique.

Cette action semble s'exercer sur les structures responsables de l'évacuation de l'humeur aqueuse. Il était donc intéressant de découvrir et d'expérimenter des analogues stéroïdiens pour déterminer ceux qui seraient le plus efficace et en particulier des dérivés du 19-nor androstane dont on pouvait penser qu'ils seraient dépourvus d'action progestéronique.

La posologie utile s'échelonne de 1 à 5 gouttes par jour dans chaque oeil d'une solution ou suspension renfermant de 0,05 à 1 % d'un principe actif de formule générale I à VII. De préférence les solutions ou suspensions renferment de 0,1 à 0,5 % de principe actif de formule générale I à VII.

Les modèles expérimentaux utilisés pour tester les composés selon l'invention sont au nombre de deux :
- modèle du glaucome expérimental chez le lapin par injection d'une solution de glucose à 5% : ce modèle décrit par BONOMI et Coll, montre que l'injection de glucose à 5% chez le lapin provoque l'élévation de la pression intra-oculaire de 8 mmHg en 5 à 10 mn, puis un retour à la normale en 40 minutes environ. Le mécanisme de cette modification de la PI0 s'explique par la réduction de l'osmolarité du sang par hémodilution ainsi qu'une diminution de la facilite d'écoulement de l'humeur aqueuse par hydratation des cellules du trabéculum.
- modèle de glaucome expérimental par injection d'alpha-chymotrypsine : on provoque le glaucome un mois avant la réalisation des tests par injection dans la chambre postérieure d'un oeil, d'une enzyme protéolytique, l'alpha-chymotrypsine. Ceci provoque la lyse de la zonule cristallinienne, une luxation du cristallin dans la chambre postérieure et une hypertension par obstruction mécanique de l'angle irido-cornéen.

Sur ces modèles, les composés selon l'invention, à la concentration de 0,1 à 0,5% provoquent une diminution rapide, importante et liée à la concentration, de la pression intra-oculaire.

Tout particulièrement,
- le 6-pivaloyloxy méthyl 17α-acetoxy 3,20-dioxo 19-nor pregna 4,6-diène
- le 6-hydroxyméthyl 17α-acétoxy 3,20-dioxo 19-nor pregna 4,6-diène
- la 17α-hydroxy progestérone
amènent une baisse importante de la pression intra-oculaire chez l'animal rendu hypertendu et les diminutions obtenues sont hautement significatives d'un point de vue statistique (p<0,01).

## Revendications

1. Utilisation pour la réalisation d'une composition pharmaceutique destinée au traitement du glaucome d'au moins un composé de structure stéroïdienne choisi dans le groupe constitué par :
A - LES 19-NOR PREGNA 4,6-DIENES DE FORMULA GENERALE I 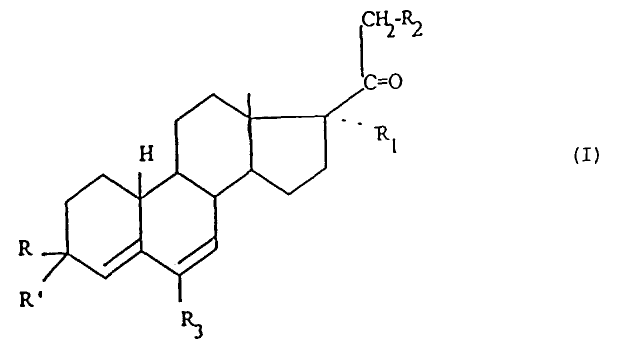 dans laquelle
R₁ représente un radical hydroxy, alcoxy, acyloxy
R₂ représente de l' hydrogène ou un radical alcoyle inférieur ayant de 1 à 4 atomes de carbone
R et R' ensemble forment un groupement cétonique, hydroxyimino, alcoxy imino ou acyloxy imino
ou bien séparément, lorsque R = H, R' est un groupement hydroxy, alcoxy ou acyloxy,
et R₃ représente un radical alcoyle inférieur ayant de 1 à 3 atomes de carbone,
B - LES 19-NOR PREGNA 4,6-DIENES DE FORMULE GENERALE II 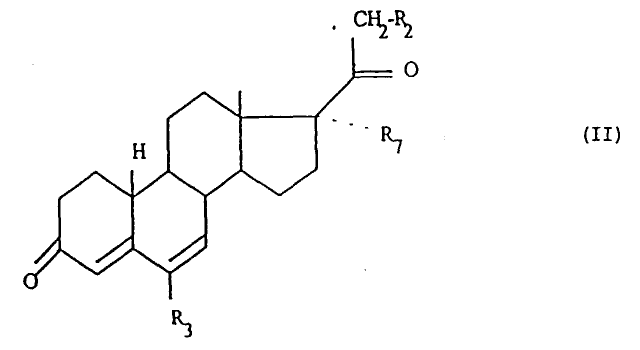 dans laquelle
R₂ représente un atome d' hydrogène ou un radical alcoyle ayant de 1 à 6 atomes de carbone, en chaîne droite ou ramifiée
R₇ représente un radical alcoyle inférieur, identique ou différent de R₂, ayant de 1 à 6 atomes de carbone, en chaîne droite ou ramifiée
et R₃ est défini comme précédemment,
C - LES COMPOSES DE STRUCTURE 19-NOR PREGNENIQUE DE FORMULE GENERALE III 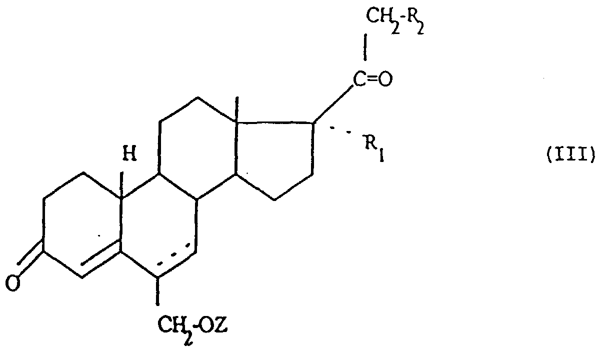 dans laquelle
R₁ représente un radical hydroxy, alcoxy, acyloxy ou alcoyle ayant de 1 à 4 atomes de carbone
R₂ représente de l' hydrogène ou un radical alcoyle inférieur en chaîne droite ou ramifiée ayant de 1 à 4 atomes de carbone
Z représente de l'hydrogène, un radical alcoyle ou un radical acyle
et le trait pointillé symbolise une double liaison carbone-carbone éventuelle,
D - LES DERIVES 19-NOR PREGNENIQUES DE FORMULE GENERALE IV 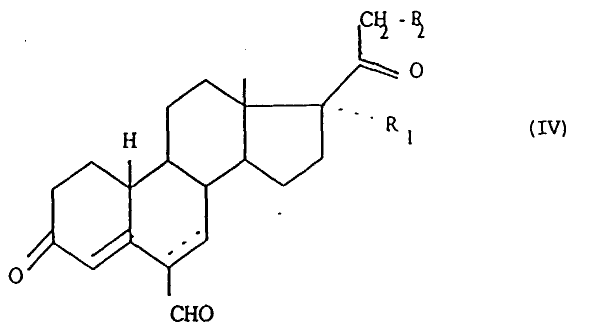 dans laquelle
R₁ représente un radical hydroxy, alcoxy, acyloxy ou alcoyle ayant de 1 à 4 atomes de carbone
R₂ représente de l' hydrogène ou un radical alcoyle inférieur en chaîne droite ou ramifiée, ayant de 1 à 4 atomes de carbone
et le trait pointillé symbolise une double liaison carbone-carbone éventuelle,
E - LES DERIVES 6-HALOGENOMETHYL 19-NOR PREGNENIQUE DE FORMULE GENERALE V 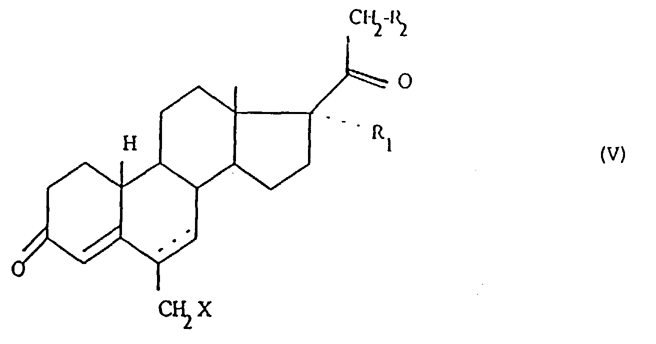 dans laquelle
X représente un atome d' halogène
R₁ et R₂ sont définis comme précédemment
et le trait pointillé symbolise une double liaison carbone-carbone éventuelle,
F - LES COMPOSES DE STRUCTURE 3 ,20 -DIOXO Δ4-PREGNENIQUE REPONDANT A LA FORMULE GENERALE VI 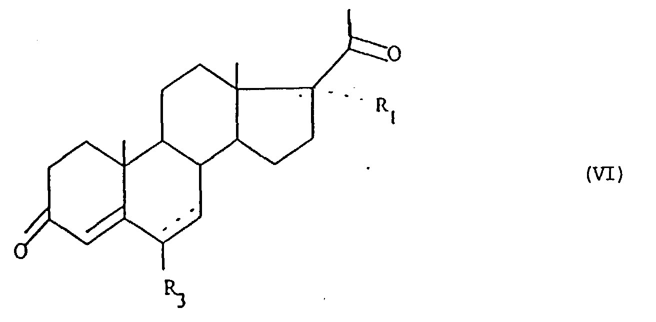 dans laquelle
R₁ représente un hydroxyle libre, éthérifié ou estérifié ou un radical alcoyle inférieur
R₃ représente de l' hydrogène ou un radical alcoyle inférieur ayant de 1 à 4 atomes de carbone
et le trait pointillé symbolise une double liaison carbone-carbone éventuelle,
G - LES COMPOSES 11,18-EPOXYANDROSTENIQUES DE FORMULE GENERALE VII 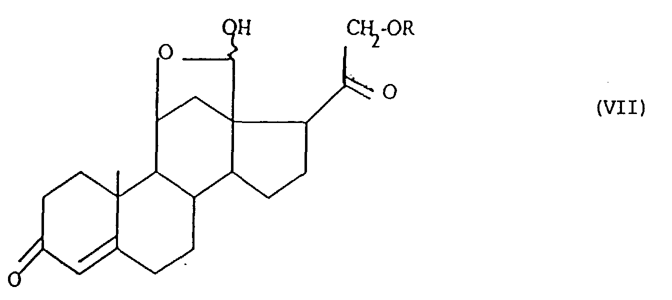
dans laquelle le trait ondulé symbolise une orientation α ou β
et R représente de l' hydrogène ou un radical acyle dérivé d' un acide organique carboxylique ayant de 1 à 10 atomes de carbone.

2. Utilisation selon la revendication 1 dans laquelle le principe actif est un 17α-hydroxy 3,20-dioxo 19-nor pregna 4,6-diène de formule Ia 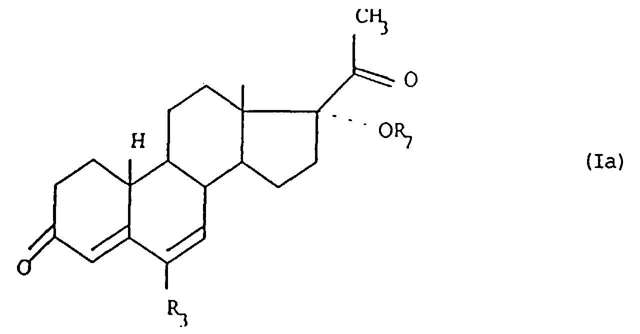
dans laquelle R₇ représente un atome d'hydrogène, un radical acyle ayant de 1 à 8 atomes de carbone
et R₃ est un radical méthyle ou propyle.

3. Utilisation selon la revendication 1 dans laquelle le principe actif est un dérivé de 17α-hydroxy 3,20-dioxo 19-nor pregna 4,6-diène de formule générale IIIa 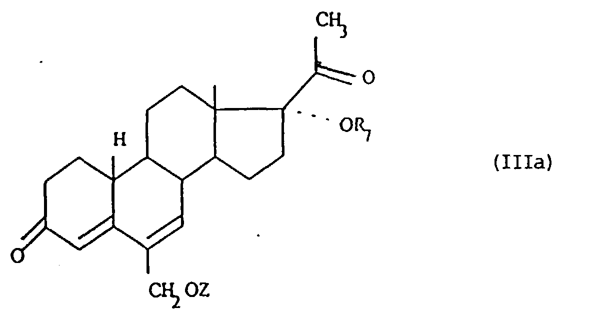 dans laquelle
R₇ représente un atome d' hydrogène, un radical alcoyle ayant de 1 à 8 atomes de carbone, un radical tétrahydropyranyl-2 ou un radical acyle dérive d'un acide carboxylique aliphatique, saturé ou non saturé, ayant de 1 à 10 atomes de carbone, éventuellement substitué par un radical aryle ou cycloalcoyle,
Z représente de l' hydrogène, un radical alcoyle ou un radical acyle.

4. Utilisation selon la revendication 1 dans laquelle le principe actif est un dérivé de 17α-hydroxy 3,20-dioxo 21-méthyl 19-nor pregna 4, 6-diène de formule générale IIIb 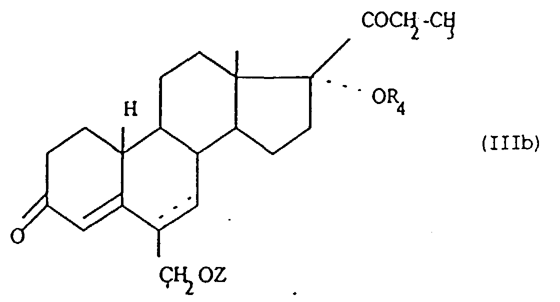 dans laquelle
R₄ représente un atome d' hydrogène, un radical alcoyle inférieur, un radical méthoxyméthyle, un radical tétrahydropyranyl-2 ou un radical acyle d'un acide organique carboxylique ayant de 1 à 10 atomes de carbone,
Z représente de l' hydrogène, un radical alcoyle ou un radical acyle.

5. Utilisation selon la revendication 1 dans laquelle le principe actif est un dérivé alcoylé de 3,20-dioxo 19-nor pregna 4,6-diène de formule générale IIIc 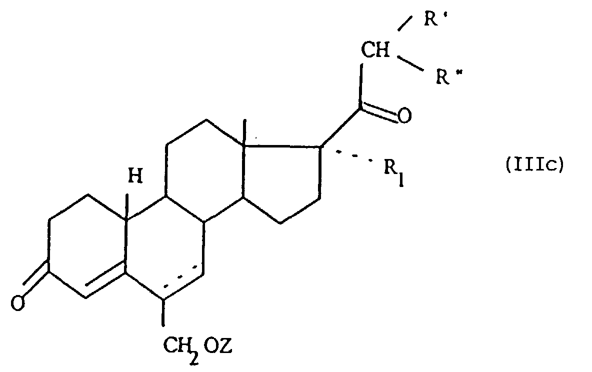 dans laquelle
R' et R'' représentent chacun un atome d'hydrogène ou un radical alcoyle ayant de 1 à 3 atomes de carbone, en chaîne linéaire ou ramifiée
R₁ représente un radical alcoyle inférieur, identique ou différent de R' et R'', ayant de 1 à 3 atomes de carbone en chaîne linéaire ou ramifiée
Z représente de l'hydrogène, un radical alcoyle ou un radical acyle.

6. Utilisation selon la revendication 1 dans laquelle le principe actif est un dérivé 19-nor pregna 4-énique de formule générale IIId 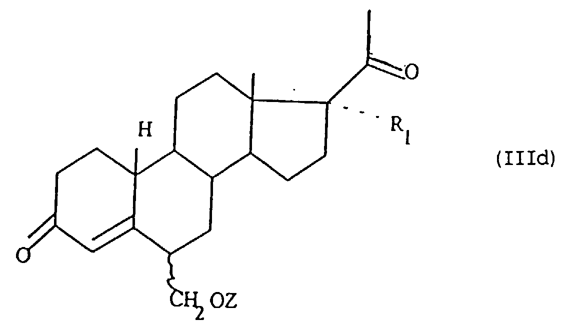 dans laquelle
R₁ est un radical alcoyle, un hydroxy, un alcoxy, un acyloxy
Z représente de l' hydrogène, un radical alcoyle ou un radical acyle
et le trait ondulé indique une orientation α ou β

7. Utilisation selon la revendication 1 ou la revendication 3 dans laquelle le principe actif est le 6-pivaloyloxy méthyl 17α-hydroxy 3,20-dioxo 19-nor pregna 4,6-diène.

8. Utilisation selon la revendication 1 ou la revendication 3 dans laquelle le principe actif est le 6-pivaloyloxy méthyl 17α-acetoxy 3,20-dioxo 19-nor pregna 4,6-diène.

9. Utilisation selon la revendication 1 ou la revendication 3 dans laquelle le principe actif est le 6-hydroxyméthyl 17α-acétoxy 3,20-dioxo 19-nor pregna 4,6-diène.

10. Utilisation selon la revendication 1 dans laquelle le principe actif est la 17α-hydroxy progestérone.

11. Utilisation selon la revendication 1 dans laquelle le principe actif est l'isoaldostérone.

12. Utilisation selon la revendication 1 dans laquelle le principe actif est le 3,20-dioxo 17α-acetoxy 6-chlorométhyl 19-nor pregna 4,6-diène de formule Va

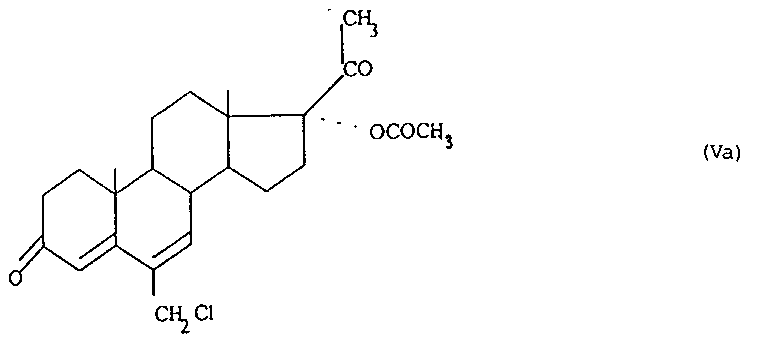

13. Utilisation selon la revendication 1 dans laquelle le principe actif est le 3,20-dioxo 17α-acetoxy 6-fluorométhyl 19-nor pregna 4,6-diène de formule Vb

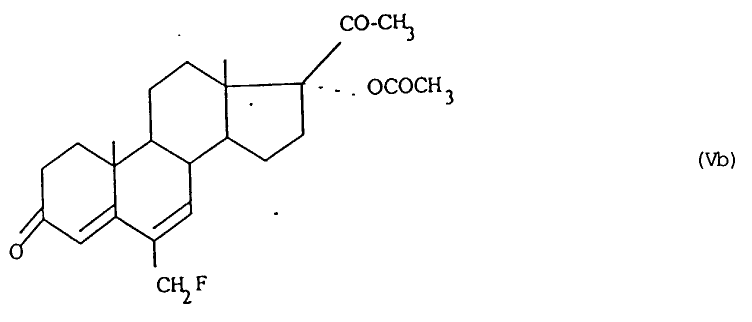

14. Utilisation selon l'une des revendications 1 à 13 dans laquelle les compositions pharmaceutiques sont sous forme solide à dissolution extemporanée, semi-solide ou liquide.

15. Utilisation selon l' une des revendications 1 à 14 dans laquelle on ajoute à la composition pharmaceutique anti-glaucomateuse un agent isotonisant aux sécrétions lacrymales.

16. Utilisation selon l'une des revendications 1 à 14 dans laquelle on ajoute à la composition pharmaceutique anti-glaucomateuse un agent de stabilisation.

17. Utilisation selon l'une des revendications 1 à 14 dans laquelle on ajoute à la composition pharmaceutique anti-glaucomateuse un agent anti-oxydant.

18. Utilisation selon l'une des revendications 1 à 17 dans laquelle la teneur en principe actif de la composition pharmaceutique selon l'une des revendications 1 à 13 s'échelonne de 0,05 à 1 %.

## Claims

1. Use for the production of a pharmaceutical composition which is intended to the treatment of glaucoma, of at least one compound with a steroïdal structure selected from the group consisting of :
A - 19-NOR PREGNA 4,6-DIENS OF THE GENERAL FORMULA I 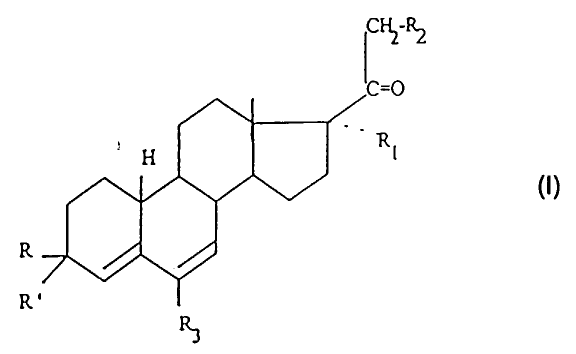 wherein
R₁ is a hydroxy, alcoxy, acyloxy radical
R₂ is a hydrogen or a lower alkyl radical having from 1 to 4 carbon atoms
R and R' form a keto, hydroxyimino, alcoxyimino or acyloxyimino group
or separadedly, when R = H, R' is an hydroxy, alcoxy or acyloxy group,
and R₃ is a lower alkyl radical having from 1 to 3 carbon atoms.
B - 19-NOR PREGNA 4,6-DIENS OF THE GENERAL FORMULA II 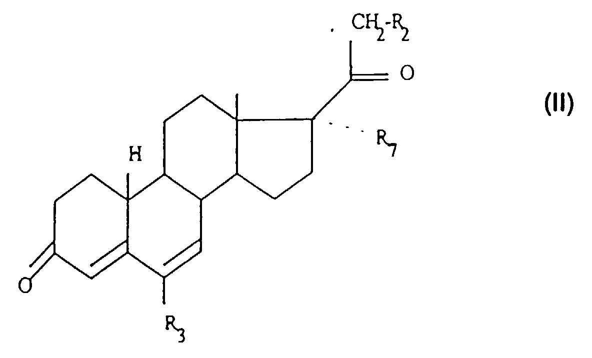 wherein
R₂ is an hydrogen atom or an alkyl radical having from 1 to 6 carbon atoms, branched or not
R₇ is a lower alkyl radical, the same or different than R₂, having from 1 to 6 carbon atoms branched or not
and R₃ is defined as previously
C - COMPOUNDS WITH A 19-NOR PREGNENIC STRUCTURE HAVING THE GENERAL FORMULA III 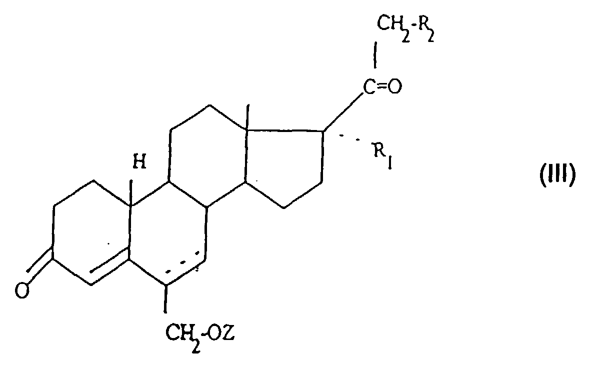 wherein
R₁ is an hydroxy, alkoxy, acyloxy or alkyl radical having from 1 to 4 carbon atoms
R₂ is a hydrogen or a lower alkyl radical branched or not, having from 1 to 4 carbon atoms
Z is a hydrogen, an alkyl or an acyl radical
and the dotted line symbolizes an optional carbon-carbon double bond,
D - 19-NOR PREGNENIC DERIVATIVES OF THE GENERAL FORMULA IV 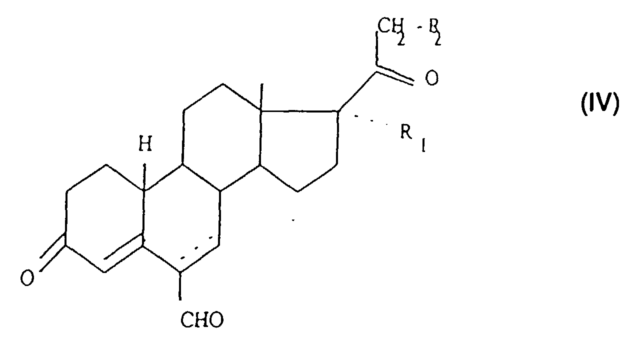 wherein
R₁ is a hydroxy, an alkoxy, an acyloxy or an alkyl radical having from 1 to 4 carbon atoms
R₂ is a hydrogen or a lower alkyl radical, branched or not, having from 1 to 4 carbon atoms
and the dotted line symbolizes an optional carbon-carbon double bond,
E - 6-HALOGENOMETHYL 19-NOR PREGNENIC DERIVATIVES OF THE GENERAL FORMULA V 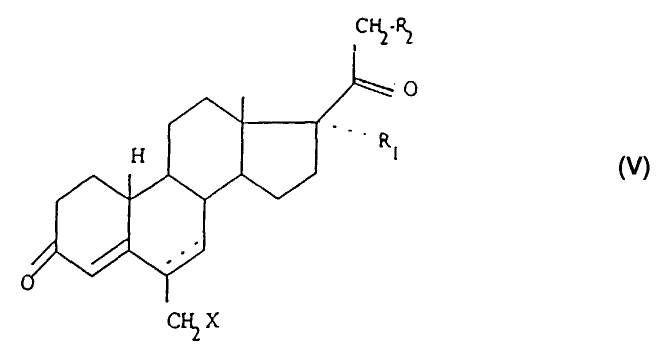 wherein
X is an halogen atom
R₁ and R₂ are defined as previously
and the dotted line symbolizes an optional carbon-carbon double bond,
F - COMPOUNDS WITH A 3,20-DIOXO Δ 4-PREGNENIC STRUCTURE WHICH CORRESPONDS TO THE GENERAL FORMULA VI 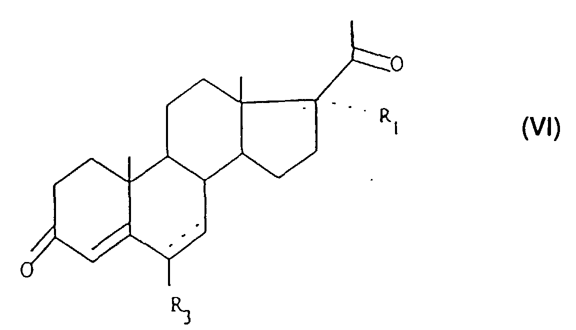 wherein
R₁ is a free, etherified, or esterified hydroxyl or a lower alkyl radical
R₃ is a hydrogen or a lower alkyl radical having from 1 to 4 carbon atoms
and the dotted line symbolizes an optional carbon-carbon double bond,
G - 11,18-EPOXYANDROSTENIC COMPOUNDS OF THE GENERAL FORMULA VII 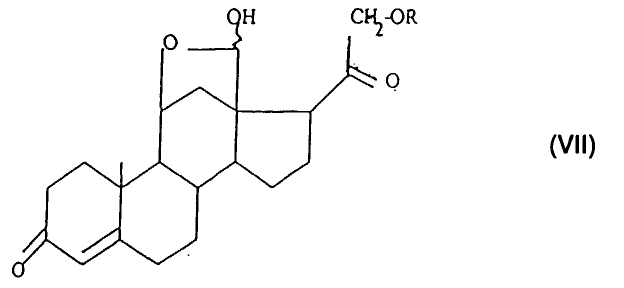
wherein the wawed line symbolizes an α or β positioning
and R is a hydrogen or an acyl radical derived from a carboxylic organic acid having from 1 to 10 carbon atoms.

2. Use according to claim 1 wherein the active agent is a 17α-hydroxy 3,20-dioxo 19-nor pregna 4,6-dien of the formula Ia 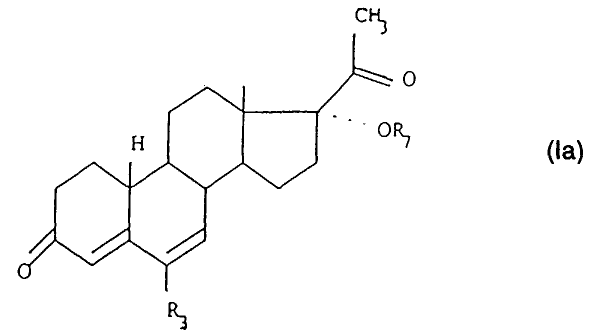
wherein R₇ is a hydrogen, an acyl radical having from 1 to 8 carbon atoms
and R₃ is a methyl or a propyl radical.

3. Use according to claim 1 wherein the active agent is a 17α-hydroxy 3,20-dioxo 19-nor pregna 4,6-dien derivative of the general formula IIIa 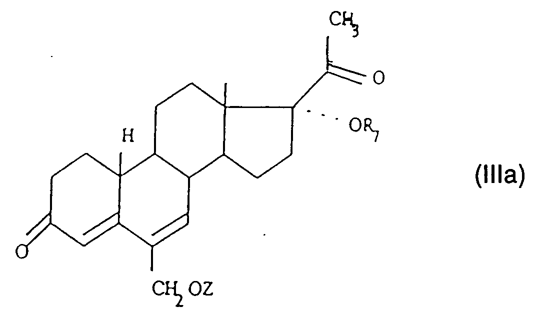 wherein
R₇ is a hydrogen, an alkyl radical having from 1 to 8 carbon atoms, a tetrahydropyranyl-2 radical or an acyl radical derived from an aliphatic carboxylic acid, saturated or unsaturated, having from 1 to 10 carbon atoms, optionaly substituted with an aryl or a cycloalkyl radical,
Z is hydrogen, an alkyl radical or an acyl radical.

4. Use according to claim 1 wherein the active agent is a 17α-hydroxy 3,20-dioxo 21-methyl 19-nor pregna 4,6-dien derivative of the general formula IIIb 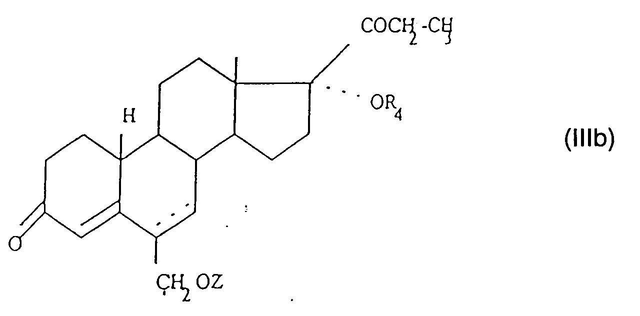 wherein
R₄ is a hydrogen, a lower alkyl radical, a methoxymethyl radical, a tetrahydropyranyl-2 radical or an acyl radical derived from an organic carboxylic acid having from 1 to 10 carbon atoms,
Z is a hydrogen, an alkyl radical or an acyl radical.

5. Use according to claim 1 wherein the active agent is an alkylated derivative from 3,20-dioxo 19-nor pregna 4,6-dien of general formula IIIc 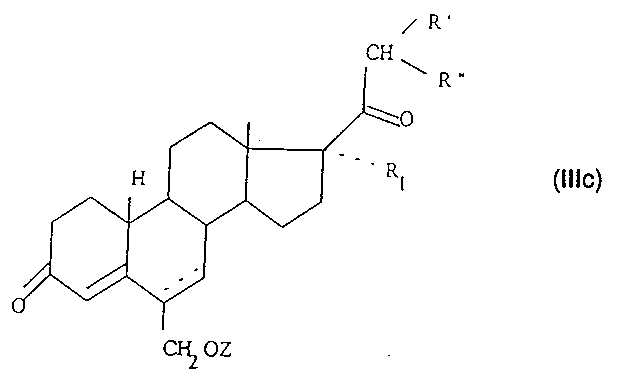 wherein
R' and R" are each other than a hydrogen or an alkyl radical having from 1 to 3 carbon atoms, branched or not
R₁ is a lower alkyl radical, the same or different than R' and R", having from 1 to 3 carbon atoms, branched or unbranched
Z is a hydrogen, an alkyl radical or an acyl radical.

6. Use according to claim 1 wherein the active ingredient is a 19-nor pregna 4-enic derivative of the general formula IIId 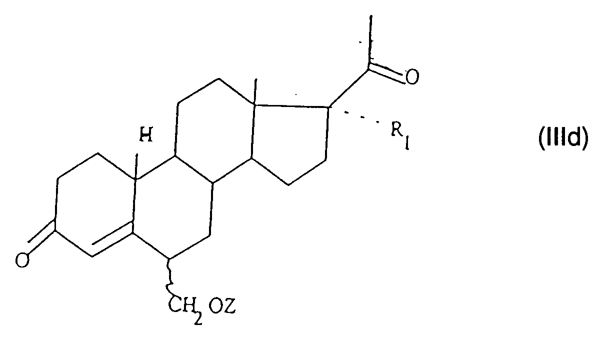 wherein
R₁ is an alkyl, hydroxy, alkoxy, acyloxy radical
Z is hydrogen, an alkyl radical or an acyl radical
and the waved line indicates an α ou β orientation.

7. Use according to claim 1 or claim 3 wherein the active ingredient is 6-(pivaloyloxy methyl) 17α-hydroxy 3,20-dioxo 19-nor pregna 4,6-dien.

8. Use according to claim 1 or claim 3 wherein the active ingredient is 6-(pivaloyloxy methyl) 17α-acetoxy 3,20-dioxo 19-nor pregna 4,6-dien.

9. Use according to claim 1 or claim 3 wherein the active ingredient is 6-(hydroxy methyl) 17α-acetoxy 3,20-dioxo 19-nor pregna 4,6-dien.

10. Use according to claim 1 wherein the active ingredient is 17α-hydroxy progesterone.

11. Use according to claim 1 wherein the active ingredient is isoaldosterone.

12. Use according to claim 1 wherein the active ingredient is 3,20-dioxo 17α-acetoxy 6-(chloromethyl) 19-nor pregna 4,6-dien of the formula Va

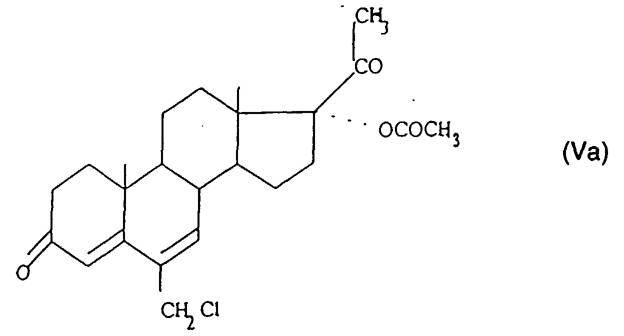

13. Use according to claim 1 wherein the active ingredient is 3,20-dioxo 17α-acetoxy 6-fluoromethyl 19-nor pregna 4,6-dien of the formula Vb.

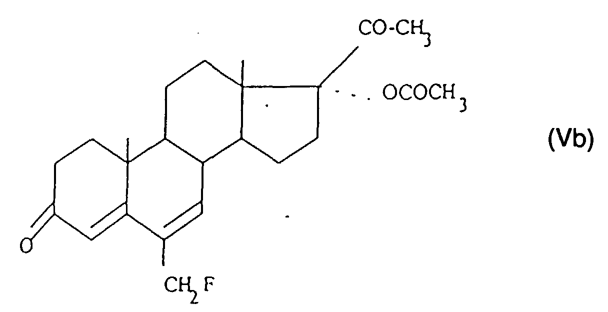

14. Use according to one of the claims 1 to 13 wherein pharmaceutical compositions are under the solid form with an immediate dissolution, semi-solid or liquid.

15. Use according to one of the claims 1 to 14 wherein an isotonizing agent to the lacrymal secretions, is added to the anti-glaucoma pharmaceutical composition.

16. Use according to one of the claims 1 to 14 wherein a stabilizing agent is added to the anti-glaucoma pharmaceutical composition.

17. Use according to one of the claims 1 to 14 wherein an anti-oxidizing agent is added to the anti-glaucoma pharmaceutical composition.

18. Use according to one of the claims 1 to 17 wherein the content of active ingredient in the pharmaceutical composition according to one of the claims 1 to 13, ranges from 0,05 % to 1 %.

## Patentansprüche

1. die Verwendung für die Herstellung einer pharmazeutische Zusammensetzung für die Behandlung des Glaukoms, mindestens einer Verbindung mit steroidischen Struktur aus der Gruppe gewählt bestehende aus :
A - 19-nor pregna 4,6-dienen der allgemeinen Formel (I) 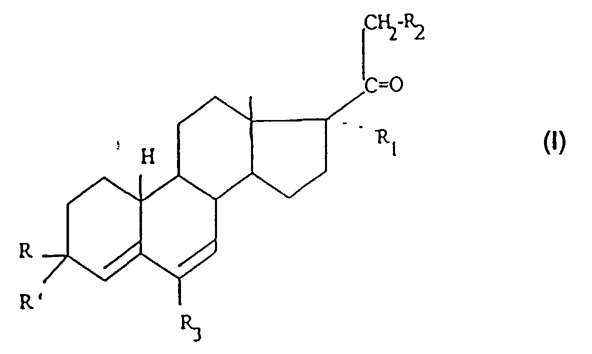 worin
R₁ für eine Hydroxy-, Alkoxy- oder Acyloxy- Rest
R₂ für eine Wasserstoff oder eine niedrige Alkyl Radikal mit 1 bis 4 Kohlenstoffatomen stehen,
R und R' zusammen eine Keto-, Hydroxymino-, Alcoxy-imino, oder Acylimino Gruppe bilden
bzw. einzeln wenn R = H, R' eine Gruppe hydroxy, alkoxy oder acyloxy ist,
und R₃ für eine niedrige Alkylrest mit 1 bis 3 Kohlenstoffatomen steht,
B - 19-nor pregna 4,6-dienen der allegemeinen Formel (II) 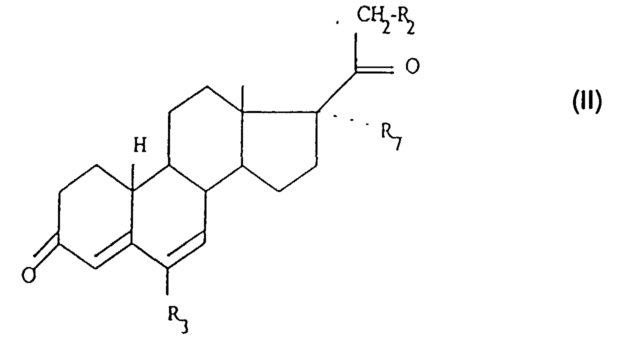 worin
R₂ für einen Wasserstoffatom, oder einen mit 1 bis 6 Kohlenstoffatomen geradekettige oder verzweigtekettige Alkylrest
R₇ für eine niedrige Alkylrest der gleich oder verschiedene als R₂ ist, und der 1 bis 6 Kohlenstoffatomen, in gerade oder verzweigte Kette hat, stehen
und R₃ wie vorher definiert ist
C - die Verbindungen mit einer 19-nor pregnenischen Struktur der allgemeinen Formel III 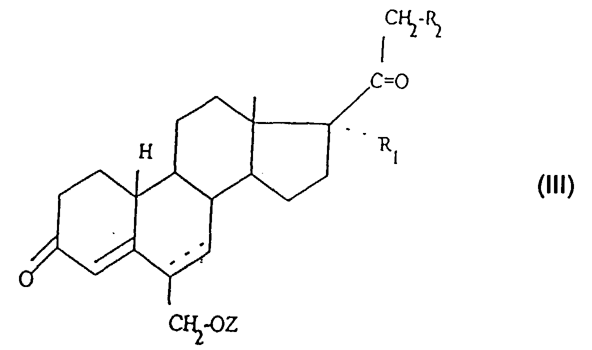 worin
R₁ für eine Hydroxy-, Alkoxy-, Acyloxy- oder Alkylgruppe mit 1 bis 4 Kohlenstoffatomen
R₂ für Wasserstoff oder eine niedrigige Alkylrest mit einer geraden oder verzweigte Kette mit 1 bis 4 Kohlenstoffatomen, hat
Z für Wasserstoff, eine Alkylrest oder eine Acylrest stehen
und die punktierte Linie eine gegebenenfalls Kohlenstoff-Kohlenstoff Doppel Bindung symboliesiert
D - Die 19-nor pregnenische Derivaten der allgemeinen Formel IV 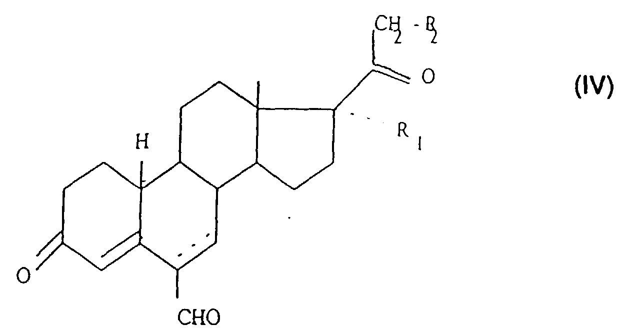 worin
R₁ für eine Hydroxy-, Alkoxy-, Acyloxy- oder Alkylrest mit 1 bis 4 Kohlenstoffatomen
R₂ für Wasserstoff, oder eine niedrige Alkyl rest mit einer geraden oder verzweigten Kette, die von 1 bis 4 Kohlenstoffatomen hat, stehen
und die punktierte Linie eine gegebenenfalls Kohlenstoff-Kohlenstoff Doppel Bindung symbolisiert,
E - Die 6-halogenomethyl 19-nor pregnenische Derivaten der allgemeinen Formel V 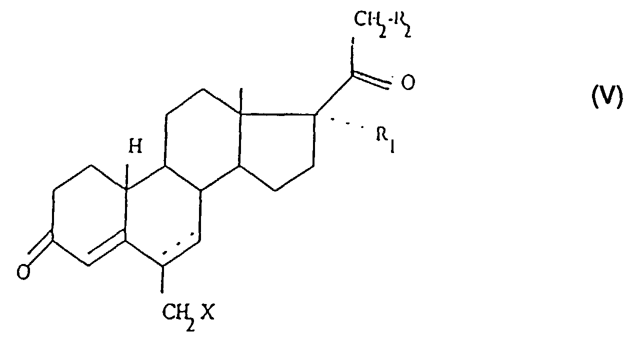 worin
X für eine Halogenatom steht
R₁ und R₂ wie vorher definiert sind
und die punktierte Linie eine gegebenfalls Kohlenstoff-Kohlenstoff Doppel Bindung symbolisiert,
F - die Verbindungen der 3,20-dioxo Δ 4-pregnenische Struktur die die allgemeine Formel VI folgen 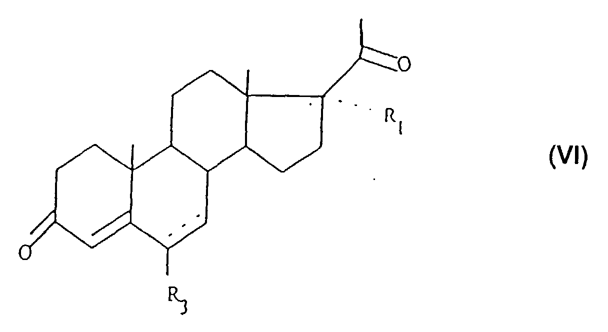 worin
R₁ für eine freie, veretherifierte, oder veresterte Hydroxy oder eine niedrige Alkyl
R₃ für Wasserstoff oder eine niedrige Alkylrest mit 1 bis 4 Kohlenstoffatomen, stehen
und die punktierte Linie eine Kohlenstoff-Kohlenstoff Doppelbindung symboliesiert,
G - Die 11,18-epoxyandrostenische Verbindungen der allgemeinen Formel VII 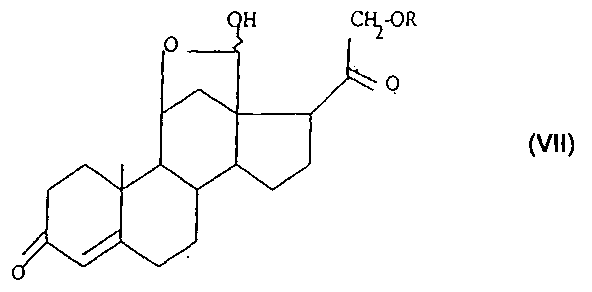
worin die gewellte Linie eine α- oder β- Richtung symboliesiert
und R für Wasserstoff oder eine Acylrest der von einer organischen Carbonsaüre mit 1 bis 10 Kohlenstoffatomen abgeleitet ist.

2. Verwendung nach Anspruch 1 worin der Wirkstoff ein 17α-Hydroxy 3,20-dioxo 19-nor pregna 4,6-dien der Formel Ia 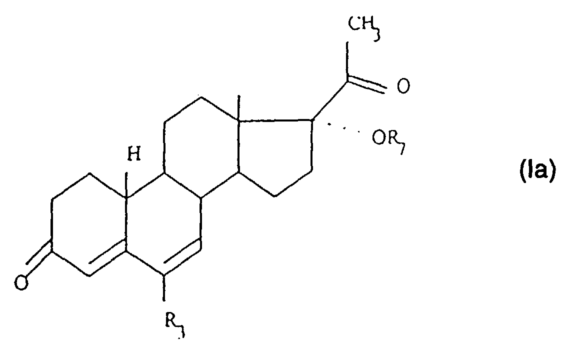 ist,
worin R₇ für eine Wasserstoffatom, ein Acylrest mit 1 bis 8 Kohlenstoffatomen steht
und R₃ eie Methyl- oder Propylradikal ist.

3. Verwendung nach Anspruch 1, worin der Wirkstoff ein Derivat des 17α-Hydroxy 3,20-dioxo 19-nor pregna 4,6-diens der allgemeinen Formel IIIa 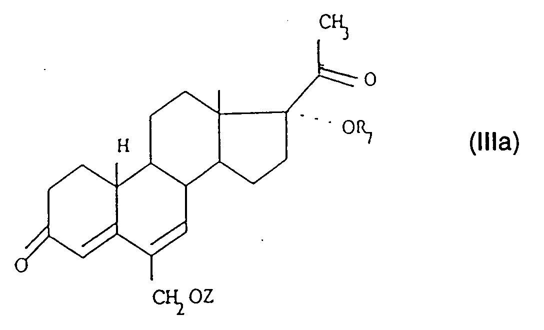 ist
worin
R₇ für eine Wasserstoffatom, eine Alkylradikal mit 1 bis 8 Kohlenstoffatomen, eine Tetrahydropyranyl-2 radikal oder eine von einer aliphatischen Carbonsaüre abgeleitete, gesättigte oder ungesättigte, gegebenfalls mit einem Acyl- oder Cycloalkylradikal substituierte, Acylrest mit 1 bis 10 Kohlenstoffatomen,
Z für Wasserstoff eine Alkylradikal oder eine Acylradikal
stehen.

4. Verwendung nach Anspruch 1, worin der Wirkstoff ein Derivat des 17α-Hydroxy 3,20-dioxo 21-methyl 19-nor pregna 4,6-diens der allgemeinen Formel IIIb 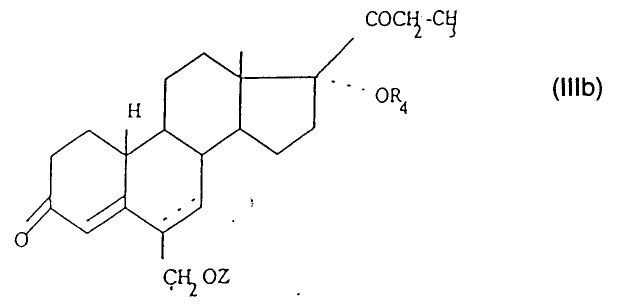 ist
worin
R₄ für eine Wasserstoffatom, eine niedrige Alkylradikal, eine Methoxymethylradikal, eine Tetrahydropyranyl-2 radikal oder den Acylrest einer organischen Carbonsaüre mit 1 bis 10 Kohlenstoffatomen,
Z für Wasserstoff, eine Alkylradikal oder eine Acylrest, stehen.

5. Verwendung nach Anspruch 1, worin der Wirkstoff eine Alkylierte Derivat des 3,20-dioxo 19-nor pregna 4,6-diens der allgemeinen Formel IIIc 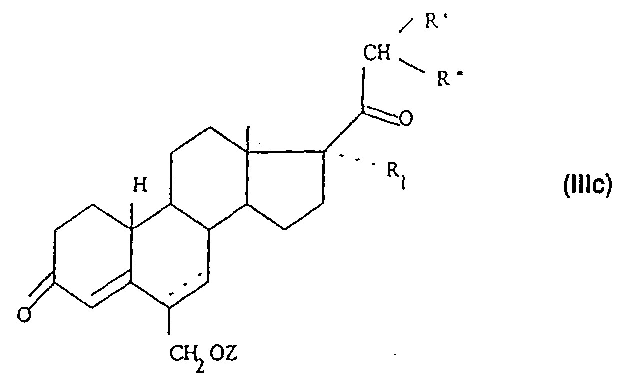 ist
worin
R' und R" peweils für eine Wasserstoffatom oder eine gerade- oder verzweigte kettige Alkylradikal mit 1 bis 3 Kohlenstoffatomen,
R₁ für eine niedrige, gleich- or verschiedene wie R' und R'', gerade kettige oder verzweigte kettige Alkylradikal, mit 1 bis 3 Kohlenstoffatomen,
Z für Wasserstoff, an Alkylradikal oder eine Acylrest, stehen.

6. Verwendung nach Anspruch 1, worin der Wirkstoff ein 19-nor pregna 4-enische Derivat der allgemeinen Formel IIId 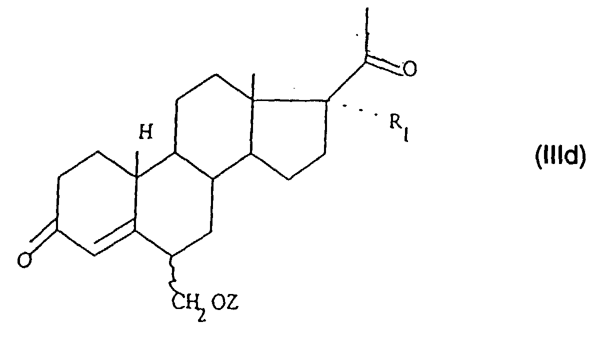 worin
R₁ ein Alkylradikal, ein Hydroxy, ein Alcoxy, ein Acyloxy ist
Z für Wasserstoff, ein Alkylradikal oder ein Acylrest steht
und die gewellte Linie eine Orientierung α oder β anzeigt.

7. Verwendung nach Anspruch 1 oder 3 worin der Wirkstoff das 6-Pivaloyloxymethyl 17α-Hydroxy 3,20-dioxo 19-nor pregna 4,6-dien ist.

8. Verwendung nach Anspruch 1 oder 3 worin der Wirkstoff das 6-Pivaloyloxymethyl 17α-acetoxy 3,20-dioxo 19-nor pregna 4,6-dien ist.

9. Verwendung nach Anspruch 1 oder 3 worin der Wirkstoff das 6-Hydroxymethyl 17α-acetoxy 3,20-dioxo 19-nor pregna 4,6-dien ist.

10. Verwendung nach Anspruch 1, worin der Wirkstoff das 17α-Hydroxy progesteron ist.

11. Verwendung nach Anspruch 1, worin der Wirkstoff das Isoaldosteron ist.

12. Verwendung nach Anspruch 1, worin der Wirkstoff das 3,20-dioxo 17α-Acetoxy 6-chlormethyl 19-nor pregna 4,6-dien der Formel Va 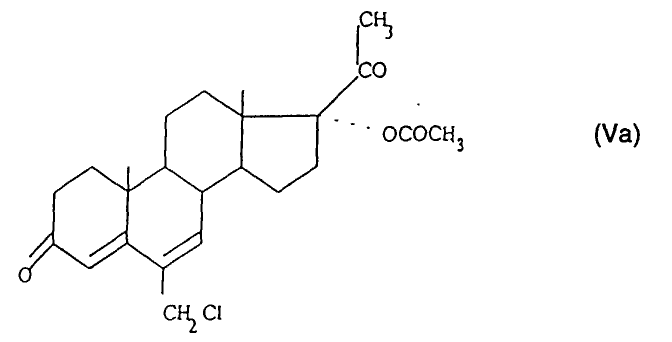 ist.

13. Verwendung nach Anspruch 1 worin der Wirkstoff das 3,20-dioxo 17α-Acetoxy 6-fluormethyl 19-nor pregna 4,6-dien der Formel Vb 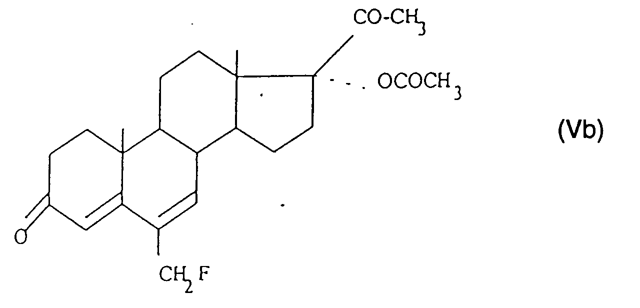 ist.

14. Verwendung nach einer der Ansprüche 1 bis 13, worin die pharmazeutischen Zusammensetzungen als feste mit sofortgefertigt Auslösung, halbfeste oder flüssige Material vorliegen.

15. Verwendung nach einer der Ansprüche 1 bis 13, worin man der pharmazeutischen Antiglaukom Zusammensetzung, eine isotonisierende Mittel für die Tränensekretionen zugibt.

16. Verwendung nach einer der Ansprüche 1 bis 14, worin man der pharmazeutischen Antiglaukom Zusammensetzung, eine stabilisierende Mittel zugibt.

17. Verwendung nach einer der Ansprüche 1 bis 14, worin man der pharmazeutischen Antiglaukom Zusammensetzung, eine Antioxydations-Mittel zugibt.

18. Verwendung nach einer der Ansprüche 1 bis 17, worin der Gehalt an Wirkstoff der pharmazeutischen Zusammensetzung nach einer der Ansprüche 1 bis 13, von 0,05 % bis 1 % sich erstreckt.
